# EUROPEAN PATENT SPECIFICATION

(11) **EP 1 689 451 B1**
(45) Date of publication and mention of the grant of the patent: **12.03.2008**
(21) Application number: 04797276.5
(22) Date of filing: 30.11.2004
(51) Int. Cl.: A61L 9/12, A01M 1/20, B60H 3/00

(54) **APPARATUS FOR EMITTING A VOLATILE LIQUID INTO THE ATMOSPHERE**
GERÄT ZUM ABGEBEN EINER FLÜCHTIGEN FLÜSSIGKEIT IN DIE ATMOSPHÄRE
APPAREIL CONCU POUR EMETTRE UN LIQUIDE VOLATILE DANS L'ATMOSPHERE

(30) Priority: 02.12.2003 US 526328 P
(43) Date of publication of application: 16.08.2006
(73) Proprietor: Givaudan SA, 1214 Vernier-Genève (CH)
(72) Inventor: MCGEE, Thomas, Nyack, NY 10960 (US); BROWN, Colin, Bracknell, Berkshire RG42 2BD (GB)
(74) Representative: McStea, John Anthony
(86) International application number: PCT/CH2004/000719
(87) International publication number: WO 2005/053756

(56) References cited:
- WO-A-03/086483
- WO-A-03/092750
- DE-A1- 19 812 022
- US-A1- 2003 091 464

## Description

This invention relates to apparatus adapted to disseminate volatile liquids into an atmosphere.

Apparatus for disseminating a volatile liquid into an atmosphere are well known. Examples of volatile liquids for dissemination include fragrances and insecticides, and atmospheres include rooms and the interiors of motor vehicles. One very common type of such apparatus consists essentially of a reservoir containing the volatile liquid and an evaporative member, such as a porous wick, one end of which contacts the liquid, the other end being exposed to the atmosphere, the liquid diffusing through the evaporative member and disseminating into the atmosphere. Generally the liquid will be present either as a straight volatile liquid or as such a liquid encapsulated in a gel. In the following description, only the case of the volatile liquid as such will be discussed, but use of the term "volatile liquid" also covers the gel embodiment.

At some convenient location (generally in a cap through which the evaporative member passes and which plugs tightly into a neck in the reservoir, otherwise keeping the liquid in the reservoir), there is a small vent, which allows the equalisation of the pressure of the reservoir with the atmosphere, ensuring that diffusion through the evaporative member will continue.

While such apparatus are generally satisfactory in normal room situations, they are not so good in atmospheres where temperatures can become very high, for example, the interior of a motor vehicle on a sunny day. In such a case, the high interior temperatures reached can cause excessive evaporation, reducing the service life considerably. Some means of overcoming this have been proposed, one being the use of an automatic cover mechanism that gradually separates the evaporative member from the atmosphere as the temperature rises, thus reducing the escape of volatile liquid into the atmosphere. Such a cover is typically operated by some temperature sensitive means (such as a bimetallic strip). This idea is useful, but it has the problem that the cover often does not make a sufficiently gas-tight seal and the loss of liquid is still high. Another approach to the problem may be found in International Publication WO 03/086483.

It has now been found that it is possible partially or completely to overcome this problem by means of a simple mechanism. The invention therefore provides an apparatus adapted to disseminate volatile liquid into an atmosphere, the apparatus comprising a reservoir containing the liquid, and a porous evaporative member that extends from the liquid into the atmosphere, the reservoir being directly open to the atmosphere only by means of a pressure equalisation vent, which vent is equipped with closing means that obstructs the vent to an increasing degree with increasing atmosphere temperature, optionally closing it completely.

The invention additionally provides a method of disseminating a volatile liquid into an atmosphere from a porous evaporative member, one end of which contacts the liquid in a reservoir that is sealed from direct contact with the atmosphere other than by a pressure equalisation vent, and the other end of which is open to the atmosphere, such that the quantity of liquid disseminated decreases with increasing temperature of the atmosphere, the method comprising the obstructing of the vent to an increasing degree with increasing temperature.

The reservoir may be any container suitable for holding a desired volatile liquid. It may be made in any suitable form and of any suitable material, plastics being especially favoured. As the porous evaporative member is the sole way for the liquid to be disseminated into the atmosphere, any construction should ensure that this is the case. A preferred construction (but by no means the only construction) is the provision of the reservoir with a relatively narrow neck, through which an essentially cylindrical evaporative member passes, the evaporative member being sealed in the neck by means of a cap that fits tightly into the neck and around the evaporative member.

The porous evaporative member may be any member suitable for conveying liquid from a reservoir by means of its internal porosity and then permitting it to evaporate into the atmosphere. It may be one of the porous wicks known to and used by the art, and it may be made of any suitable material (such as fibres, carbon, ceramics, plastics) by any suitable means (such as extrusion and moulding).

The pressure equalisation vent may be in any appropriate location. In the case mentioned above, it may be in the cap sealing the porous evaporative member into the reservoir. However, other constructions are possible. The vent is covered in some way during transport of the apparatus and the covering is removed when the apparatus is put into service. This is generally done by having a cap that covers both vent and that end of the porous evaporative means that, in service, is exposed to the atmosphere.

The vent is equipped with closing means that obstructs the vent to an increasing degree with increasing atmospheric temperature. This means that, as the temperature rises, the effective size of the vent becomes smaller, the ability of the apparatus to equalise the pressure is reduced and the quantity of liquid able to flow up the porous evaporative member is also reduced. If desired, the closing means can close the vent completely, almost completely preventing any escape of liquid into the atmosphere.

The closing means comprises a temperature-responsive moving member, and a closure member attached thereto.

The temperature-responsive moving member may be any suitable member known to the art that, as the temperature rises, causes the closure member to move to obstruct the vent to an increasing extent. For simplicity and cheapness, it is preferred that it comprise a member that deforms under increasing temperature, such that the closure member moves in an appropriate vent-restricting direction. Preferably the temperature-responsive moving member will be a single component that deforms with rising temperature to a degree sufficient to give the desired degree of closure.

An example of such a member is a coil spring. Such springs are known to expand as temperature rises, and this property can be utilised. However, the preferred temperature-responsive moving member is a bimetallic member. This is a member made by fusing together two metals with different coefficients of thermal expansion. As the temperature rises, the different rates of expansion will cause the element to deform. The most common bimetallic member is the bimetallic strip, an elongate strip made of two metal strips of equal length fused together. On heating, this strip bends towards the side with the metal of lower coefficient of thermal expansion. Thus, in the present case, such a strip may be orientated with respect to the vent such that the metal with the lower coefficient of thermal expansion is nearer the vent, with one end fixed and the other end free to move, this free end having attached thereto a closure member. As the strip bends, it brings the closure member closer to the vent, optionally eventually sealing it completely. A further possibility is a bimetallic coil, which uncoils with increasing temperature, thus expanding along the longitudinal axis of the coil.

The closure member may be any suitable closure member, and the skilled person will readily be able to conceive of many suitable closure members for any given case. For example, the closure member may be a conical member that, with rising temperature, is moved towards a circular vent, in the manner of a needle valve. As the conical member more closely approaches the vent, it reduces the area of the vent, and therefore the possibility of equalisation of pressure inside and outside the reservoir, to an ever greater degree, optionally eventually sealing it completely.

Alternatively, the closure member may be a cap that is moved towards and optionally eventually completely closes a vent. The vent may terminate at its upper end in a hole that is coplanar with a surface on which it terminates, or it may terminate in a raised seat, standing above the surface. A further alternative is a cupped member, which is moved towards a vent, such that the cup ever more obstructs, and optionally eventually seals completely, the vent, which again may be just a hole or part of a raised seat. A further possibility is an annular closure member that moves towards a corresponding annular channel formed, for example, in a sealing cap of a reservoir, the vent extending from the bottom of the channel to the interior of the reservoir. If complete sealing is desired, that part of the closure member making contact with the channel may be equipped with a member or material suitable for forming a good seal, for example, a gasket of a suitably elastomeric material such as a rubber or a plastics material. The skilled person will be able to see many possible variants that lie within the scope of this invention.

The invention is further described with reference to the accompanying drawings. These depict preferred embodiments and do not limit the invention in any way.
Figure 1 is a longitudinal cross-section of a first preferred embodiment of the invention.
Figure 2 is a longitudinal cross-section of a second preferred embodiment of the invention.
Figure 3 is a longitudinal cross-section of a third preferred embodiment of the invention.
Figure 4 is a longitudinal cross-section of a fourth preferred embodiment of the invention.
Figure 5 is a longitudinal cross-section of a fifth preferred embodiment of the invention.

A fragrance dispenser generally indicated as 1 consists of two body parts, an upper part 2 and a lower reservoir part 3, the reservoir containing a volatile liquid fragrance to be disseminated into the atmosphere. Into a neck 4 of the reservoir is fitted a porous wick 5, this being held in place by an insert 6 that tightly fits into the neck and tightly around the wick. This insert includes a vent 7, which is the only direct contact of the liquid in the reservoir with the atmosphere and which serves to equalise the air pressure. In this particular embodiment, the upper end of the vent 7 widens as it approaches the top of the insert. (Depending on the embodiment, such a widening may not always be necessary or desirable). Immediately above this widened end of the vent is located a conical plug 8, whose profile matches that of the widened end. This conical plug is located at the end of a bimetallic strip 9, which is riveted to a protrusion 10 in the wall of the upper body part 2.

The bimetallic strip and conical plug are located such that a rise in temperature will cause the strip to bend towards the neck 4, thus causing the conical plug to move into the widened end of the vent 7, thus constricting the flow of air, inhibiting the equalisation of pressure and slowing the rate of evaporation from the wick 5. Thus, in high temperatures (such as the interior of a motor vehicle on a hot day), the fragrance will not all be lost. As the temperature drops, the conical plug will withdraw, thus permitting increased evaporation again.

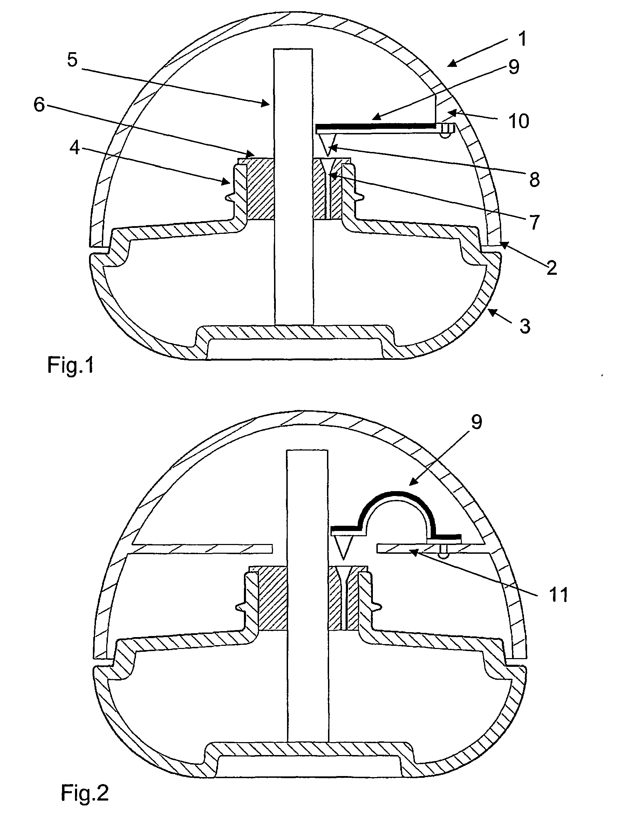

The embodiment of Figure 2 closely resembles that of Figure 1, the differences being that the protrusion 10 of figure 1 is replaced by an annular shelf 11 to which the bimetallic strip 9 is riveted, and that the strip is curved rather than straight - this allows the use of more bimetallic material in the same space and therefore more movement at the free end.

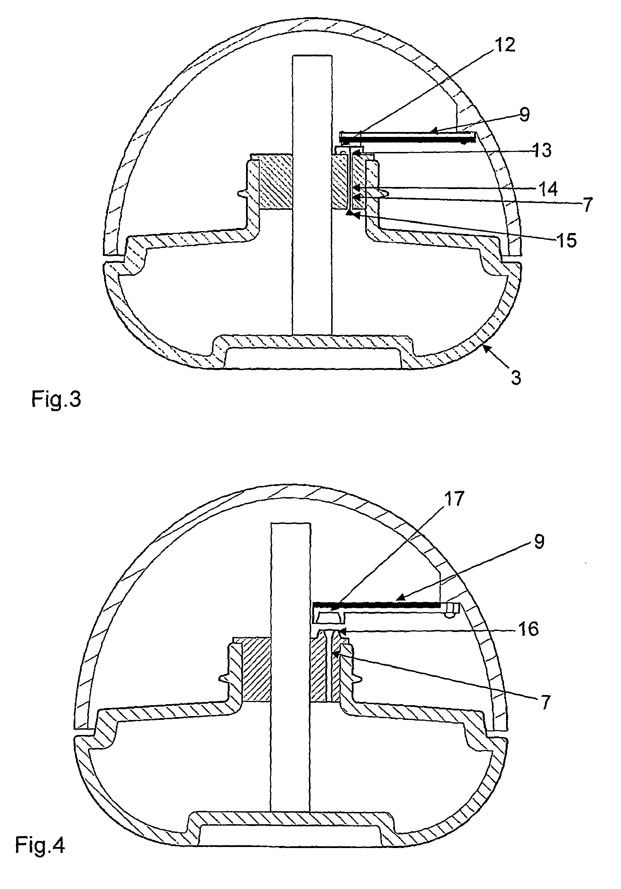

The embodiment of Figure 3 is different in that the bimetallic strip 9 bends away from the neck 4 when the temperature rises. At normal temperatures, a cap 12 at the end of the bimetallic strip 9 rests on the insert 6 that holds the wick 5 in place. The cap is positioned over a vent 7 that allows equalisation of pressure with the atmosphere in the reservoir 3. The cap is hollow and has apertures 13, to permit equalisation.

In the embodiment of Figure 3, partial or complete closing of the vent is achieved by means of a rod 14 that depends from the cap 12 through the vent 7, and which has at its lower end a conical member 15. This conical member is adapted to constrict the flow of air from the atmosphere to the reservoir 3, and if desired to close it off completely, by the fact that, as the bimetallic strip bends upward under increasing temperature, it pulls the rod and the associated conical member closer to the lower opening of the vent.

The embodiment of Figure 4 is essentially the same as that of Figure 1, but in this case, the upper end of the vent 7 is raised to form a neck 16 and the conical plug 8 is replaced by a cup 17 designed to fit over the neck 16 and reduce, and if desired completely cut off the reservoir from the atmosphere.

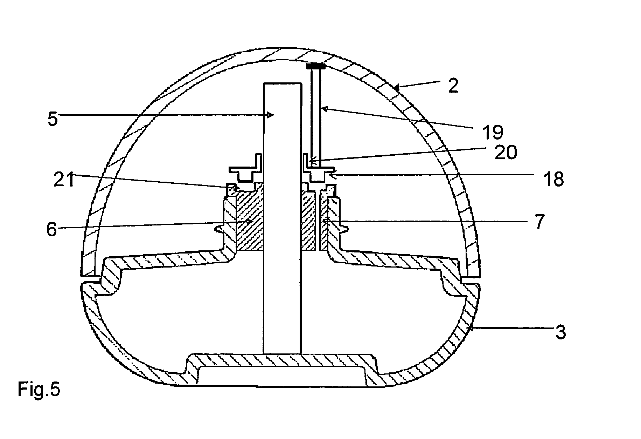

In the embodiment of Figure 5, the closure member is an annular member 18, which is suspended from the upper body part 2 by means of a bimetallic coil 19. The annular closure member is formed with a collar 20 through which protrudes a porous wick 5, the collar allowing the closure member to move slidably on the wick 5. The wick is held in the reservoir 3 by means of an insert 6 that fits tightly into a neck 4 of the reservoir. On the upper surface of this insert is formed a channel 21, this being dimensioned and positioned such that the closure member will fit therein. From this channel through the insert to the reservoir runs a vent 7. If complete sealing is desired, the bottom of the annular closure member 18 typically comprises a rubber gasket.

In operation, the bimetallic coil 19 expands with rising temperature and moves the annular closure member 18 in the direction of the channel 21. As it moves, it gradually cuts off the contact of the reservoir with the atmosphere, thus reducing the ability of the volatile liquid in the reservoir to travel up the wick. If desired, the annular member can be configured to close the vent completely.

## Claims

1. An apparatus (1) adapted to disseminate volatile liquid into an atmosphere, the apparatus comprising a reservoir (2, 3) containing the liquid, and a porous evaporative member (5) that extends from the liquid into the atmosphere, the reservoir being directly open to the atmosphere only by means of a pressure equalisation vent (7), which vent is equipped with closing means comprising a temperature-responsive moving member (9), and a closure member (8, 15, 17, 18) attached thereto, the closing means being adapted to obstruct the vent (7) to an increasing degree with increasing atmosphere temperature, optionally closing it completely.

2. An apparatus according to claim 1, in which the temperature-responsive moving member comprises a member that deforms under increasing temperature, such that the closure member moves in an appropriate vent-restricting direction.

3. An apparatus according to claim 2, in which the temperature-responsive moving member is a single component that deforms with rising temperature to a degree sufficient to give the desired degree of closure.

4. An apparatus according to claim 3, in which the temperature-responsive moving member is a coil spring.

5. An apparatus according to claim 3, in which the temperature-responsive moving member is a bimetallic member.

6. An apparatus according to claim 5, in which the bimetallic member is selected from a bimetallic strip and a bimetallic coil.

7. An apparatus according to claim 1, in which the closure member is a needle valve (8) that fits in a circular vent.

8. An apparatus according to claim 1, in which the closure member is a cap (17) that closes an orifice.

9. A method of disseminating a volatile liquid into an atmosphere from a porous evaporative member, one end of which contacts the liquid in a reservoir that is sealed from direct contact with the atmosphere other than by a pressure equalisation vent, and the other end of which is open to the atmosphere, such that the quantity of liquid disseminated decreases with increasing temperature of the atmosphere, the method comprising the obstructing of the vent to an increasing degree with increasing temperature by means of a temperature-responsive moving member and a closure member attached thereto.

## Patentansprüche

1. Gerät zur Dissemination einer volatilen Flüssigkeit in eine Atmosphäre, enthaltend einen Vorratsbehälter (2, 3), der die Flüssigkeit enthält, und ein poröses Verdampfungselement (5), das sich aus der Flüssigkeit in die Atmosphäre erstreckt, wobei der Vorratsbehälter nur durch einen Druckausgleichskanal (7) direkt in die Atmosphäre offen ist, der mit einer Schließeinrichtung versehen ist, die ein auf Temperatur ansprechendes, bewegliches Element (9) und ein daran befestigtes Schließelement (8, 15, 17, 18) umfasst, wobei die Schließeinrichtung dazu eingerichtet ist, den Kanal (7) bei ansteigender Temperatur zunehmend und ggf. vollständig zu verschließen.

2. Gerät nach Anspruch 1, bei dem das auf Temperatur ansprechende, bewegliche Element ein solches Element umfasst, das sich bei steigender Temperatur so verformt, dass das Schließelement sich in eine geeignete, den Kanal verengende Richtung bewegt.

3. Gerät nach Anspruch 2, bei dem das auf Temperatur ansprechende, bewegliche Element ein einziges Bauteil ist, das sich bei steigender Temperatur in einem Ausmaß verformt, das ausreichend groß ist, das gewünschte Maß an Schließwirkung zu ergeben.

4. Gerät nach Anspruch 3, bei dem das auf Temperatur ansprechende, bewegliche Element eine Schraubenfeder ist.

5. Gerät nach Anspruch 3, bei dem das auf Temperatur ansprechende, bewegliche Element ein Bimetall-Element ist.

6. Gerät nach Anspruch 5, bei dem das Bimetall-Element ein Bimetallstreifen oder eine Bimetallspule ist.

7. Gerät nach Anspruch 1, bei dem das Schließelement ein Nadelventil (8) ist, das in einen kreisförmigen Kanal passt.

8. Gerät nach Anspruch 1, bei dem das Schließelement eine Kappe (17) ist, die eine Öffnung verschließt.

9. Verfahren zur Dissemination einer volatilen Flüssigkeit in eine Atmosphäre aus einem porösen Verdampfungselement, von dem ein Ende die Flüssigkeit in einem Vorratsbehälter berührt, der gegen einen direkten Kontakt mit der Atmosphäre mit Ausnahme eines Druckausgleichskanals abgeschlossen ist, und das andere Ende zur Atmosphäre hin offen ist, so dass die Menge an verdampfter Flüssigkeit mit steigender Temperatur der Atmosphäre abnimmt, wobei das Verfahren umfasst: Versperren des Kanals in einem zunehmenden Maße bei Ansteigen der Temperatur mittels eines auf Temperatur ansprechenden, beweglichen Elements und eines daran befestigten Schließelements.

## Revendications

1. Appareil (1) conçu pour diffuser un liquide volatil dans l'atmosphère, l'appareil comprenant un réservoir (2, 3) contenant le liquide, et un élément (5) d'évaporation poreux qui s étend à partir du liquide dans l'atmosphère, le réservoir étant directement ouvert à l'atmosphère uniquement par l' intermédiaire d' un évent (7) d'égalisation de pression, lequel évent est équipé de moyens de fermeture comprenant un organe mobile (8) sensible à la température et un organe de fermeture (8, 15, 17, 18) fixé à celui-ci, les moyens de fermeture étant prévus pour obstruer l'évent (7) à un degré croissant lorsque la température de l'atmosphère augmente, éventuellement jusqu'à le fermer complètement.

2. Appareil selon la revendication 1, dans lequel l'organe mobile sensible à la température comprend un élément qui se déforme lorsque la température augmente de telle sorte que l'organe de fermeture se déplace dans une direction appropriée de réduction de l'évent.

3. Appareil selon la revendication 2, dans lequel l'organe mobile sensible à la température est un composant unique qui se déforme lorsque la température augmente à un degré suffisant pour assurer le degré désiré de fermeture.

4. Appareil selon la revendication 3, dans lequel l'organe mobile sensible à la température est un ressort hélicoïdal.

5. Appareil selon la revendication 3, dans lequel l'organe mobile sensible à la température est un élément bilame.

6. Appareil selon la revendication 5, dans lequel l'élément bilame est choisi entre une bande bimétallique et une bobine bimétallique.

7. Appareil selon la revendication 1, dans lequel l'organe de fermeture est une soupape à pointeau (8) qui s'ajuste dans un évent circulaire.

8. Appareil selon la revendication 1, dans lequel l'organe de fermeture est un capuchon (17) qui ferme un orifice.

9. Procédé de diffusion d'un liquide volatil dans une atmosphère à partir d'un élément d'évaporation poreux, dont une extrémité est en contact avec le liquide dans un réservoir isolé de tout contact direct avec l'atmosphère sauf par un évent d'égalisation de pression, et dont l'autre extrémité est ouverte à l'atmosphère, de telle sorte que la quantité de liquide diffusée diminue lorsque la température de l'atmosphère augmente, le procédé comprenant le fait d'obstruer l'évent à un degré croissant au fur et à mesure de l'augmentation de la température au moyen d'un organe mobile sensible à la température et d'un organe de fermeture fixé à celui-ci.
